# EUROPÄISCHE PATENTSCHRIFT

(11) **EP 3 772 347 B1**
(45) Veröffentlichungstag und Bekanntmachung des Hinweises auf die Patenterteilung: **15.06.2022**
(21) Anmeldenummer: 20189311.2
(22) Anmeldetag: 04.08.2020
(51) Int. Cl.: A61B 17/86, A61B 50/30, A61C 8/00, B65D 75/32, B65D 75/58

(54) **VERFAHREN ZUM HERSTELLEN EINER VERPACKUNG UND VERPACKUNG**
METHOD FOR MANUFACTURING A PACKAGING AND PACKAGING
PROCÉDÉ DE FABRICATION D'UN EMBALLAGE ET EMBALLAGE

(30) Priorität: 05.08.2019 DE 102019121111
(43) Veröffentlichungstag der Anmeldung: 10.02.2021
(73) Patentinhaber: Medipack AG, 8200 Schaffhausen (CH)
(72) Erfinder: ARTUSI, Reto, 8200 Schaffhausen (CH)
(74) Vertreter: Patentanwälte und Rechtsanwalt Weiß, Arat & Partner mbB

(56) Entgegenhaltungen:
- WO-A1-2010/065980
- WO-A1-2018/187824
- DE-A1- 2 707 841
- JP-A- 2012 116 514
- JP-U- S5 533 265
- US-A- 4 155 454
- US-A1- 2017 283 145

## Beschreibung

### Technisches Gebiet

Die Erfindung betrifft ein Verfahren zum Herstellen einer Verpackung für eine Knochenschraube, wobei in einen Streifen zumindest eine Kavität für eine Knochenschraube eingeformt, die Knochenschraube in die Kavität eingelegt und die Kavität von einem Deckstreifen verschlossen wird, sowie eine diesbezügliche Verpackung.

### Stand der Technik

Verfahren zum Herstellen einer Verpackung für zumindest einen Gegenstand sowie derartige Verpackungen selbst sind in vielfältiger Form und Ausführung bekannt und auf dem Markt. Im vorliegenden Fall geht es vor allem um die Verpackung von Gegenständen, welche bei medizinischen Eingriffen Anwendung finden sollen, speziell um Knochenschrauben. Diese Knochenschrauben sind in der Regel sehr scharfzahnig, wodurch die Gefahr besteht, dass die Verpackung beschädigt wird. Ferner unterliegen sie erheblichen hygienischen Anforderungen und sollten möglichst wenig dem Zugriff der ungeschützten menschlichen Hand ausgesetzt sein.

Aus der EP 3 153 128 A1 ist bspw. eine Verpackungshülse für medizinische Zwecke zur Aufbewahrung von sterilen Gegenständen bekannt. Diese besteht aus einer einseitig offenen und mit einem Dichtverschluss verschliessbaren Aussenhülse, in der eine Innenhülse klemmend aufgenommen ist, wobei die Innenhülse im Klemmsitz in einer bodenseitigen Aufnahmeöffnung des Dichtverschlusses der Aussenhülse aufgenommen ist und der dadurch gebildete Klemmsitz die am Aussenumfang des Dichtverschlusses angeordneten Dichtrippen unter verstärktem Anpressdruck an die Innenseite der Aussenhülse anpresst. Diese Verpackungshülse ist sehr kompliziert aufgebaut und schwierig zu bedienen.

Ferner zeigt die DE 10 2012 112 945 A1 eine medizinische Sterilverpackung, welche einen Aufnahmeraum für mindestens ein Implantat definiert. Der Aufnahmeraum ist in einer Lagerstellung steril und keimdicht verschlossen und kann bei Nichtgebrauch während eines chirurgischen Eingriffs einfach und sicher wieder für einen zukünftigen Eingriff aufbereitet werden. Die Sterilverpackung ist aus einem oder mehreren sterilisierbaren Materialien hergestellt. Auch bei dieser Sterilverpackung ist sowohl Herstellung als auch Verwendung kompliziert, ferner ist nur eine Einzelverpackung möglich.

Auch in der DE 10 2013 019 452 A1 wird eine Einzelverpackung für langgestreckte Gegenstände, wie z.B. Knochennägel oder Knochenschraube, beschrieben. Diese besteht aus einer mindestens einseitig stirnseitig offenen, etwa zylindrischen Verpackungshülse aus einem elastischen biegbaren Material. Sie weist eine obere Einführöffnung zum Einführen des langgestreckten Gegenstandes auf, wobei die Einzelverpackung lediglich den Kopf des zu haltenden Gegenstandes in einem sich an die Einführöffnung in axialer Richtung anschliessenden, elastisch aufweitbaren Klemmbund lagengesichert hält und das bolzenseitige Ende des Gegenstandes abstandshaltend von der Innenseite der Kunststoffverpackung entfernt gehalten ist. Durch die Einhaltung dieses Abstandes wird eine Verletzung der Verpackungshülse vermieden. Allerdings ist auch hier wiederum die Herstellung, Befüllung und Handhabung dieser Einzelverpackung sehr kompliziert.

Vor diesem Hintergrund sind zudem folgende Druckschriften anzuführen:
In der DE 27 07 841 A1 wird eine Schmierfettkapsel und ein Verfahren zur Herstellung einer Schmierfettkapsel genannt, wobei eine Schmierfettkapsel bestehend aus einem Formteil mit einer Vertiefung und einer Abdeckfolie offenbart wird.

In der US 4 155 454 A wird eine Sicherheitspackung für eine Ampulle, die eine Trägerkarte, eine Deckfolie und eine Ampulle umfasst offenbart. Die Ampulle und das Abdeckblatt sind so ausgelegt, dass sie bei der Ausübung von Druck auf die Trägerkarte zerbrechen.

In der US 2017 283 145 A1 wird ein Kunststoff-Fläschchen zur Abgabe von hochviskosen oder halbfesten Materialien offenbart, welches aus zwei Platten aus thermoformbarem Kunststoff gebildet wird. Die Platten werden thermisch verformt, um ein erhöhtes Profil zu bilden, das einen Hohlraum definiert.

In der JP S55 033 265 U werden ferner unterschiedliche Verpackungen offenbart.

In der WO 2018 187 824 A1 wird ein Behälter mit zu öffnenden Deckel offenbart.

In der JP 2012 116 514 A wird zu dem ein Verpackungsmaterial für pharmazeutische Präparate und dergleichen offenbart, wobei ein konkaver Gehäuseabschnitt zur Aufnahme eines festen oder eines pulverförmigen pharmazeutischen Produkts vorgesehen ist, und ein plattenartiges Element zum Verschließen einer Öffnung des Gehäuseabschnitts angeordnet ist. Schließlich ist noch die WO 2010 065 980 A1 anzuführen. Hier wird ein Behälter mit einem Hohlraum zur Aufnahme von abgebbaren Hohlrauminhalten offenbart, wobei eine Öffnung, eine Abdeckung zum Abdecken der Öffnung; und einen Deckel, der mit dem Körper durch eine Bruchzone verbunden ist und im Gebrauch nach einem Bruch der Bruchzone um ein durch die Abdeckung gebildetes Scharnier geöffnet werden kann, angeordnet sind.

### Aufgabe der Erfindung

Die Aufgabe der vorliegenden Erfindung ist es, eine sowohl in Herstellung als auch Handhabung sehr einfache Verpackung zu entwickeln.

### Lösung der Aufgabe

Zur Lösung der Aufgabe führt, dass nach der Einformung der Kavität eine Stanzlinie in zumindest einen Teil der Wandung der Kavität eingeformt wird.

Dieser Stanzlinie kommt im Rahmen der Erfindung eine grosse Bedeutung zu. Sie dient, wie später noch beschrieben wird, dem Öffnen der Kavität zur Entnahme der Knochenschraube.

In vielen Fällen kann es gewünscht sein, dass die Kavität gänzlich verschlossen bleibt. In diesem Fall wird die Stanzlinie nicht durch die gesamte Dicke der Wandung der Kavität eingeschnitten, sondern nur zum Teil, so dass sie aufgebrochen werden kann. In einem anderen Fall wird aber bevorzugt, dass die Stanzlinie zumindest zum Teil offen ist, damit ein Sterilisiermedium an die Knochenschraube gelangen kann, bevor die Verpackung aufgebrochen und die Knochenschraube benutzt wird. In diesem Fall kann die Stanzlinie in ihrer wesentlichen Länge durchgeformt und lediglich ein oder mehrere Bruchstege stehen gelassen werden. Auch für die Form der Stanzlinie gibt es keine Begrenzung, falls gewünscht kann sie auch schlangen- oder auch zickzackartig verlaufen.

Um das Abbiegen eines Teils der Verpackung bzw. das Öffnen der Stanzlinie zu erleichtern, soll einem Randstreifen, der die Kavität umläuft, eine Knicklinie eingeformt sein. Zur Erleichterung der gesamten Stanzschritte könnte es wünschenswert sein, dass diese Knicklinie zusammen mit der Stanzlinie erzeugt wird. Von der Lage her bietet es sich an, dass die Knicklinie von der Stanzlinie ausgehend nach aussen zum Rand des Randstreifens hin verläuft. Auch für die Knicklinie kann vorgesehen sein, dass sie durch das Material des Randstreifens durchgestanzt ist oder nur teilweise in das Material des Randstreifens eingestanzt wird.

Bevorzugt soll diese Verpackung nicht als Einzelverpackung hergestellt und angeboten werden, sondern als Blister, d. h., in der Anordnung einer Mehrzahl von Verpackungen neben- bzw. übereinander. Zum Gebrauch kann dann eine Einzelverpackung aus dem Blister herausgebrochen und einer weiteren Verwendung zugeführt werden. Um das Ausbrechen zu erleichtern, werden um die Einzelverpackungen herum jeweils Bruchlinien ausgeformt. Dies ist aber aus Blisterverpackungen bspw. für Medikamente allgemein bekannt.

Bevorzugt wird sowohl für den Streifen, aus dem Kavitäten und die Randstreifen herausgeformt werden, als auch für den Deckstreifen Folien aus Polyurethan verwendet. Dieses Polyurethan hat den Vorteil, dass es auch durch scharfzahnige Gegenstände, wie bspw. Knochenschrauben, nicht verletzt wird. Des Weiteren ist es insbesondere zur Verwendung in einem Autoklaven sehr geeignet, da es relativ hohe Temperaturen aushält.

Vom Erfindungsgedanken wird auch eine Verpackung mit einer Knochenschraube umfasst, wobei in einen Streifen zumindest eine Kavität für die zumindest eine Knochenschraube eingeformt, die Knochenschraube in die Kavität eingelegt und die Kavität von einem Deckstreifen verschlossen ist, und wobei in zumindest einen Teil der Wandung der Kavität eine Stanzlinie eingeformt ist. Diese Stanzlinie kann wie oben beschrieben ausgebildet sein, insbesondere ist sie aber bevorzugt offen und lediglich von zumindest einem Bruchsteg unterbrochen.

Des Weiteren ist vorgesehen, dass in einen Randstreifen, der die Kavität zumindest teilweise umläuft, eine Knicklinie eingeformt ist, wobei die Knicklinie bevorzugt seitlich von der Stanzlinie nach aussen zu einer Randkante des Randstreifens verläuft.

Für die Ausgestaltung der Kavität kann es wünschenswert sein, dass diese, bspw. wenn insbesondere Schrauben verpackt werden sollen, einen sich halsartig verjüngenden Bereich aufweist, wobei in diesem Fall die Stanzlinie in den halsartig verjüngten Bereich eingeformt ist. Erfindungsgemäß ist die Wandung der Kavität mit Versteifungsrippen versehen, welche auch bei Druckbeaufschlagung eine Zurückformung der Kavität in die gewünschte Ausgangslage gewährleisten.

Bevorzugt kann an beliebiger Stelle der Verpackung, insbesondere aber oberhalb der Stanzlinie, eine Lasche vorgesehen werden, welche ein Abknicken zumindest eines Teils der Verpackung und damit ein Öffnen der Stanzlinie gewährleisten. Diese Lasche kann auch der Beschriftung dienen, bspw. der Beschreibung des Inhalts der Verpackung.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in:
Figur 1 eine Draufsicht auf einen Blister, zusammengesetzt aus einer Mehrzahl von erfindungsgemässen Verpackungen;
Figur 2 eine Seitenansicht des Blisters gemäss Figur 1;
Figur 3 eine vergrössert dargestellte Draufsicht auf eine erfindungsgemässe Verpackung in Schliesslage;
Figur 4 eine Draufsicht auf die Verpackung gemäss Figur 3 in aufgebrochener Lage;
Figur 5 eine Darstellung des erfindungsgemässen Verfahrensablaufs zur Herstellung einer erfindungsgemässen Verpackung.

### Ausführungsbeispiel

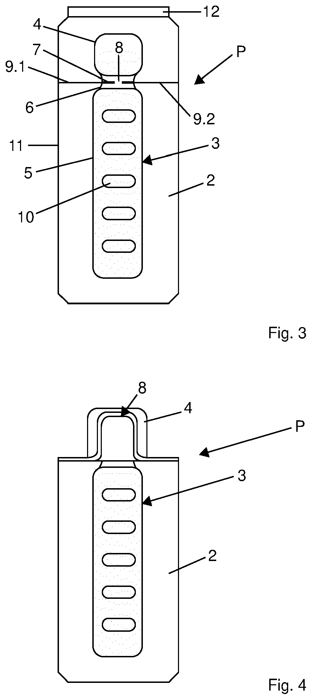

Gemäss den Figuren 3 und 4 weist eine erfindungsgemässe Verpackung P eine Knochenschraube 1 und einen Randstreifen 2 auf, der eine Kavität 3 umläuft. Diese Kavität 3 ist flaschenartig ausgebildet, wobei zwischen einem Kopfbereich 4 und einem Körper 5 ein halsartiger, verjüngter Bereich 6 vorgesehen ist. In diesen halsartigen Bereich 6 ist eine Stanzlinie 7 eingeformt, die durch einen Bruchsteg 8 unterbrochen ist.

Des Weiteren ist in den Randstreifen 2, anschliessend an die Stanzlinie 7 jeweils eine Knicklinie 9.1 bzw. 9.2 eingeformt, welche, ausgehend von der Stanzlinie 7 nach aussen zum äusseren Rand 11 des Randstreifens 2 verlaufen.

Erfindungsgemäß sind in dem Körper 5 der Kavität 3 noch Versteifungsrippen 10 eingeformt, die beliebige geometrische Ausformungen aufweisen können.

An den Randstreifen 2 ist, bevorzugt oberhalb des Kopfbereichs 4 der Kavität 3, noch eine Lasche 12 angeformt, welche der Handhabung der Verpackung P insbesondere beim Aufbrechen des Bruchstegs 8 dient, aber auch für eine Beschreibung der Knochenschraube 1 dienen kann.

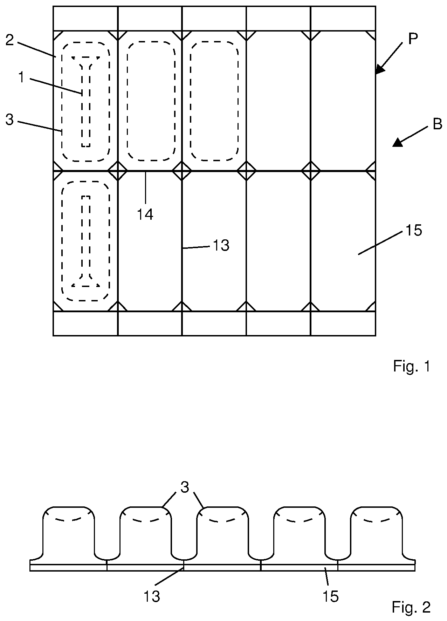

Bevorzugt werden eine Mehrzahl von Verpackung P, wie in den Figuren 1 und 2 gezeigt, zusammen als Blister B angeordnet. In diesem Fall werden in einen entsprechenden Materialstreifen eine Mehrzahl von Randstreifen 2 ausgeformt, wobei benachbarte Randstreifen 2 von entsprechenden Bruchlinien 13 bzw. 14 voneinander abgegrenzt werden.

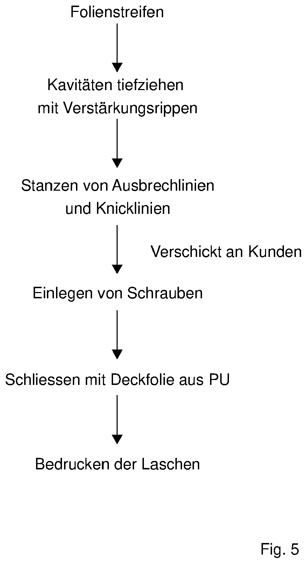

Die Funktionsweise der vorliegenden Erfindung ist folgende:
Die Herstellung der erfindungsgemässen Verpackung P bzw. des Blisters B erfolgt folgendermassen und ist in Figur 5 stichwortartig beschrieben. Als erstes wird ein Blisterstreifen im Falle der Herstellung eines Blisters aus einer Mehrzahl von Verpackungen oder ein Streifen im Falle der Herstellung einer einzelnen Verpackung P bereitgestellt. Bevorzugt soll dieser Streifen sowohl für den Blister als auch für die einzelne Verpackung aus Polyurethan bestehen und folienartig ausgebildet sein. In diesen Streifen wird dann eine Kavität oder eine Mehrzahl von Kavitäten 3 durch Tiefziehen eingeformt. Diese Kavität 3 hat eine gewünschte geometrische Ausgestaltung, die sich vor allem auch nach der Form der zu verpackenden Knochenschraube 1 richtet. Hier ist erfindungsgemäss an jede beliebige Form gedacht. In dieser Herstellungsstufe können auch z.B. die Versteifungsrippen 10 mit eingeformt werden.

Nunmehr erfolgt die Einformung der Stanzlinie 7 mit dem stehenbleibenden Bruchsteg 8 sowie der Knicklinien 9.1 und 9.2.

In diesem Zustand kann nun die Verpackung P oder der Blister B mit der Knochenschraube 1 befüllt oder an einen Kunden verschickt werden, der die Verpackung P bzw. den Blister B mit der Knochenschraube 1 befüllt.

Nach dem Einlegen der Knochenschraube 1 wird der Blister B bzw. die Verpackung P durch einen Deckstreifen 15 verschlossen, welcher bevorzugt ebenfalls aus Polyurethan besteht. Ferner wird, falls gewünscht, die Lasche 12 mit einer Beschreibung der Knochenschraube 1 bedruckt.

Im Falle des Herstellens eines Blisters erfolgt nun die Einformung der Bruchlinien 13 und 14 in den Blister B, wobei entsprechende Verbindungsstege stehen bleiben, die das Ausbrechen einer einzelnen Verpackung P aus dem Blister B erlauben.

Zur Entnahme der Knochenschraube 1 wird die einzelne Verpackung P in einer Hand gehalten, die sich im Bereich des Körpers 5 befindet. Mit den Fingern der anderen Hand wird die Lasche 12 ergriffen und, wie insbesondere in Figur 3 gezeigt, über die Knicklinie 9.1 bzw. 9.2 abgebogen. Dabei bricht der Bruchsteg 8 und die Stanzlinie 7 öffnet sich, so dass die Knochenschraube 1 einem Zugriff offenliegt.

**Bezugszeichenliste**

| | | | |
|---|---|---|---|
| 1 | Knochenschraube | | |
| 2 | Randstreifen | | |
| 3 | Kavität | | |
| 4 | Kopfbereich | | |
| 5 | Körper | | |
| 6 | halsartiger Bereich | | |
| 7 | Stanzlinie | | |
| 8 | Bruchsteg | | |
| 9 | Knicklinie | | |
| 10 | Versteifungsrippe | | |
| 11 | Rand | | |
| 12 | Lasche | | |
| 13 | Bruchlinie | | |
| 14 | Bruchlinie | | |
| 15 | Deckstreifen | | |
| 16 | | B | Blister |
| 17 | | | |
| 18 | | | |
| 19 | | | |
| 20 | | P | Verpackung |
| 21 | | | |
| 22 | | | |
| 23 | | | |
| 24 | | | |
| 25 | | | |
| 26 | | | |
| 27 | | | |
| 28 | | | |
| 29 | | | |
| 30 | | | |
| 31 | | | |
| 32 | | | |
| 33 | | | |

## Patentansprüche

1. Verfahren zum Herstellen einer Verpackung (P) für eine Knochenschraube (1), wobei in einen Streifen zumindest eine Kavität (3) für die Knochenschraube (1) eingeformt, die Knochenschraube (1) in die Kavität (3) eingelegt und die Kavität (3) von einem Deckstreifen (15) verschlossen wird,
wobei, nach der Einformung der Kavität (3) mit Versteifungsrippen (10), eine Stanzlinie (7) in zumindest einen Teil der Wandung der Kavität (3) eingeformt wird, wobei die Stanzlinie (7) von zumindest einem Bruchsteg (8) unterbrochen wird.

2. Verfahren nach Anspruch 1, **dadurch gekennzeichnet, dass** in einen Randstreifen (2), der die Kavität (3) zumindest teilweise umläuft, eine Knicklinie (9.1,9.2) eingeformt wird.

3. Verfahren nach Anspruch 2, **dadurch gekennzeichnet, dass** die Knicklinie (9.1,9.2) jeweils seitlich von der Stanzlinie (7) nach außen zu einer Randkante (11) des Randstreifens (2) verläuft.

4. Verfahren nach wenigstens einem der vorhergehenden Ansprüche, **dadurch gekennzeichnet, dass** eine Mehrzahl von Kavität (3) mit umlaufenden Randstreifen (2) in den Streifen eingeformt und um die Randstreifen Bruchlinien (13,14) ausgebildet werden.

5. Verfahren nach wenigstens einem der vorhergehenden Ansprüche, **dadurch gekennzeichnet, dass** für den Streifen mit der zumindest einen Kavität (3) und/oder für den Deckstreifen (15) eine Folie aus Polyurethan verwendet wird.

6. Verpackung mit zumindest einer Knochenschraube (1), wobei in einen Streifen zumindest eine Kavität (3) für die Knochenschraube (1) eingeformt, die Knochenschraube (1) in der Kavität (3) eingelegt und die Kavität (3) von einem Deckstreifen (15) verschlossen ist, wobei in zumindest einen Teil der Wandung der Kavität (3) eine Stanzlinie (7) eingeformt ist, wobei die Kavität (3) Versteifungsrippen (10) aufweist und wobei die Stanzlinie (7) von zumindest einem Bruchsteg (8) unterbrochen ist.

7. Verpackung nach Anspruch 6, **dadurch gekennzeichnet, dass** in einen Randstreifen (2), der die Kavität (3) zumindest teilweise umläuft, eine Knicklinie (9.1,9.2) eingeformt ist.

8. Verpackung nach Anspruch 7, **dadurch gekennzeichnet, dass** die Knicklinie (9.1,9.2) seitlich von der Stanzlinie (7) nach außen zu einer Randkante (11) des Randstreifens (2) verläuft.

9. Verpackung nach Anspruch 7 oder 8, **dadurch gekennzeichnet, dass** der Deckstreifen (15) im Bereich der Knicklinie (7) unversehrt ist.

10. Verpackung nach wenigstens einem der Ansprüche 6 bis 9, **dadurch gekennzeichnet, dass** die Kavität (3) einen halsartig verjüngten Bereich (6) aufweist.

11. Verpackung nach Anspruch 10, **dadurch gekennzeichnet, dass** die Stanzlinie (7) in den halsartig verjüngten Bereich (6) eingeformt ist.

12. Verpackung nach wenigstens einem der Ansprüche 6 bis 11, **dadurch gekennzeichnet, dass** der Kavität (3) eine Lasche (12) zum Beschriften und/oder Erleichtern des Öffnens der Stanzlinie (7) zugeordnet ist.

## Claims

1. Method for producing a package (P) for a bone screw (1), wherein in a strip at least one cavity (3) for the bone screw (1) is formed, the bone screw (1) is inserted into the cavity (3) and the cavity (3) is closed by a cover strip (15), wherein, after the cavity (3) has been formed with stiffening ribs (10), a stamp line (7) is formed in at least part of the wall of the cavity (3), wherein the stamp line (7) is interrupted by at least one breaking web (8).

2. Method according to claim 1, **characterized in that** in an edge strip (2), which at least partially surrounds the cavity (3), a knuckle line (9.1,9.2) is formed.

3. Method according to claim 2, **characterized in that** the knuckle line (9.1, 9.2) in each case runs laterally from the stamp line (7) outwards to an edge (11) of the edge strip (2).

4. Method according to at least one of the preceding claims, **characterized in that** a plurality of cavities (3) with circumferential edge strips (2) are formed in the strip and break lines (13, 14) are formed around the edge strips.

5. Method according to at least one of the preceding claims, **characterized in that** for the strip with the at least one cavity (3) and/or for the cover strip (15) a film made of polyurethane is used.

6. Packaging with at least one bone screw (1), wherein in a strip at least one cavity (3) for the bone screw (1) is formed, the bone screw (1) is inserted in the cavity (3) and the cavity (3) is closed by a cover strip (15), wherein in at least one part of the wall of the cavity (3) a stamp line (7) is formed, wherein the cavity (3) has stiffening ribs (10) and wherein the stamp line (7) is interrupted by at least one breaking web (8).

7. Packaging according to claim 6, **characterized in that** in an edge strip (2), which at least partially surrounds the cavity (3), a knuckle line (9.1,9.2) is formed.

8. Packaging according to claim 7, **characterized in that** the knuckle line (9.1, 9.2) runs laterally outwards from the stamp line (7) to an edge (11) of the edge strip (2).

9. Packaging according to claim 7 or 8, **characterized in that** the cover strip (15) is intact in the area of the stamp line (7).

10. Packaging according to at least one of claims 6 to 9, **characterized in that** the cavity (3) has a neck-like tapered region (6).

11. Packaging according to claim 10, **characterized in that** the stamp line (7) is formed into the neck-like tapered region (6).

12. Packaging according to at least one of claims 6 to 11, **characterized in that** the cavity (3) is assigned a flap (12) for labeling and/or facilitating the opening of the stamp line (7).

## Revendications

1. Procédé de fabrication d'un emballage (P) destiné à une vis à os (1), au moins une cavité (3) destinée à la vis à os (1) étant formée dans une bande, la vis à os (1) étant placée dans la cavité (3) et la cavité (3) étant fermée par une bande de revêtement (15), une ligne de découpe (7), après le formage de la cavité (3) au moyen de nervures de rigidification (10), étant formée dans au moins une partie de la paroi de la cavité (3), la ligne de découpe (7) étant interrompue par au moins une barrette de rupture (8).

2. Procédé selon la revendication 1, **caractérisé en ce qu'**une ligne de pliage (9.1, 9.2) est formée dans une bande de bordure (2) qui s'étend au moins partiellement autour de la cavité (3).

3. Procédé selon la revendication 2, **caractérisé en ce que** la ligne de pliage (9.1, 9.2) s'étend respectivement latéralement vers l'extérieur depuis la ligne de découpe (7) jusqu'à une bordure (11) de la bande de bordure (2).

4. Procédé selon au moins l'une des revendications précédentes, **caractérisé en ce que** plusieurs cavités (3) comportant des bandes de bordure (2) périphériques sont formées dans la bande et des lignes de rupture (13, 14) sont conçues autour des bandes de bordure.

5. Procédé selon au moins l'une des revendications précédentes, **caractérisé en ce qu'**un film de polyuréthane est utilisé pour la bande comportant l'au moins une cavité (3) et/ou pour la bande de revêtement (15).

6. Emballage comportant au moins une vis à os (1), au moins une cavité (3) destinée à la vis à os (1) étant formée dans une bande, la vis à os (1) étant placée dans la cavité (3) et la cavité (3) étant fermée par une bande de revêtement (15), une ligne de découpe (7) étant formée dans au moins une partie de la paroi de la cavité (3), la cavité (3) présentant des nervures de rigidification (10) et la ligne de découpe (7) étant interrompue par au moins une barrette de rupture (8).

7. Emballage selon la revendication 6, **caractérisé en ce qu'**une ligne de pliage (9.1, 9.2) est formée dans une bande de bordure (2) qui s'étend au moins partiellement autour de la cavité (3).

8. Emballage selon la revendication 7, **caractérisé en ce que** la ligne de pliage (9.1, 9.2) s'étend latéralement vers l'extérieur depuis la ligne de découpe (7) jusqu'à une bordure (11) de la bande de bordure (2).

9. Emballage selon la revendication 7 ou 8, **caractérisé en ce que** la bande de revêtement (15) est intacte dans la zone de la ligne de pliage (7).

10. Emballage selon au moins l'une des revendications 6 à 9, **caractérisé en ce que** la cavité (3) présente une zone (6) qui se rétrécit à la manière d'un goulot.

11. Emballage selon la revendication 10, **caractérisé en ce que** la ligne de découpe (7) est formée dans la zone (6) qui se rétrécit à la manière d'un goulot.

12. Emballage selon au moins l'une des revendications 6 à 11, **caractérisé en ce qu'**une languette (12) permettant de marquer et/ou de faciliter l'ouverture de la ligne de découpe (7) est associée à la cavité (3).
